(19) Europäisches Patentamt
European Patent Office
Office européen des brevets

(11) Veröffentlichungsnummer : **0 343 182 B1**

# (12) EUROPÄISCHE PATENTSCHRIFT

(45) Veröffentlichungstag der Patentschrift :
**06.05.92 Patentblatt 92/19**

(21) Anmeldenummer : **88901412.2**

(22) Anmeldetag : **04.02.88**

(86) Internationale Anmeldenummer :
**PCT/EP88/00081**

(87) Internationale Veröffentlichungsnummer :
**WO 88/05770 11.08.88 Gazette 88/18**

(51) Int. Cl.$^5$ : **C07C 33/20,** C07C 29/14, C07C 29/17, A61K 7/46, C07C 29/40, C07C 29/36

## (54) ALKOHOLE MIT 3-METHYL- ODER 3,5-DIMETHYLPHENYLGRUPPEN, DEREN HERSTELLUNG UND VERWENDUNG ALS DUFTSTOFFE.

(30) Priorität : **06.02.87 DE 3703584**

(43) Veröffentlichungstag der Anmeldung :
**29.11.89 Patentblatt 89/48**

(45) Bekanntmachung des Hinweises auf die Patenterteilung :
**06.05.92 Patentblatt 92/19**

(84) Benannte Vertragsstaaten :
**CH DE FR GB LI NL**

(56) Entgegenhaltungen :
**EP-A- 0 032 659**
**EP-A- 0 076 493**
**EP-A- 0 217 159**

(73) Patentinhaber : **Consortium für elektrochemische Industrie GmbH**
**Zielstattstrasse 20**
**W-8000 München 70 (DE)**

(72) Erfinder : **HAFNER, Walter**
**Birkenallee 17**
**W-8196 Eurasburg (DE)**
Erfinder : **GEBAUER, Walter**
**Schaffhauser Str. 18/7**
**W-8000 München 71 (DE)**
Erfinder : **REGIERT, Marlies**
**Schraudolphstrasse 2 A**
**W-8000 München 40 (DE)**
Erfinder : **FRIEDRICH, Wilhelm**
**Ernst-Platz-Strasse 34/0**
**W-8000 München 50 (DE)**
Erfinder : **MARKL, Erich**
**Terofalstrasse 41/III**
**W-8000 München 21 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Alkohole mit 3-Methyl- oder 3,5-Dimethylphenylgruppen, deren Herstellung und Verwendung als Duftstoffe

3-Phenylpropionaldehyd und 3-Phenylpropan-1-ol werden seit langem als Duftstoffe verwendet. Weiterhin ist der Duftstoffcharakter von Propionaldehyden der allgemeinen Formel

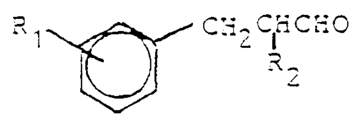

aus der japanischen Offenlegungsschrift JP 61,194,045 (CA 106:32752m) bekannt.

Aus der EP-A-0076493 ist die Verwendung von 1,1-Di($C_1$-$C_6$-alkyl)-2-phenyl-ethan-Derivaten als Riechstoffe bekannt. Alle dort beschriebenen Verbindungen weisen jedoch keine zusätzliche Substitution des Phenylrestes auf, wie beispielsweise für 2,2-Dimethyl-3-phenylpropanol in Beispiel 2 dieser Schrift ersichtlich.

Es wurde nun gefunden, daß durch Methylsubstitution in 3- oder 3,5-Stellung des Benzylrestes neue Duftstoffe mit überwiegend blumigem Charakter entstehen.

Gegenstand der Erfindung sind 2-Methyl- 3-(3-methylphenyl)-propan-1-ol, 2-Methyl-3-(3,5-dimethylphenyl)-propan-1-ol, 1-(3-Methylphenyl)-2-methylbutan-3-ol und 1-(3,5-Dimethylphenyl)-2-methylbutan-3-ol.

Ein Verfahren zur Herstellung von 2-Methyl-3-(3-methylphenyl)-propan-1-ol und 2-Methyl-3-(3,5-dimethylphenyl)-propan-1-ol ist dadurch gekennzeichnet, daß

a) 3-Methylbenzaldehyd oder 3,5-Dimethylbenzaldehyd mit Propionaldehyd in Gegenwart von Basen umgesetzt wird und

b) das Reaktionsprodukt gemäß a) hydriert wird, oder

a1) 3-Methylbenzylmagnesiumchlorid oder 3,5-Dimethylbenzylmagnesiumchlorid mit Chloraceton umgesetzt wird und

b1) das Reaktionsprodukt gemäß a1) nach basenkatalysierter Cyclisierung katalytisch hydriert wird.

Diese Reaktionswege werden an folgenden Schemata verdeutlicht.

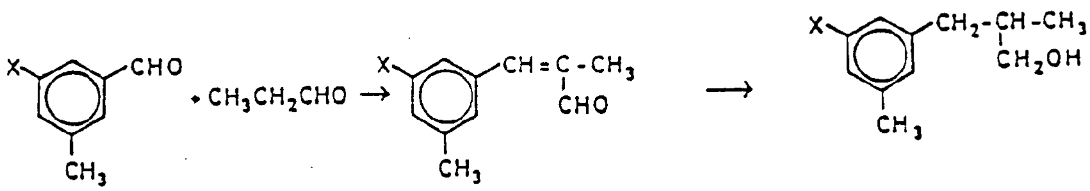

oder

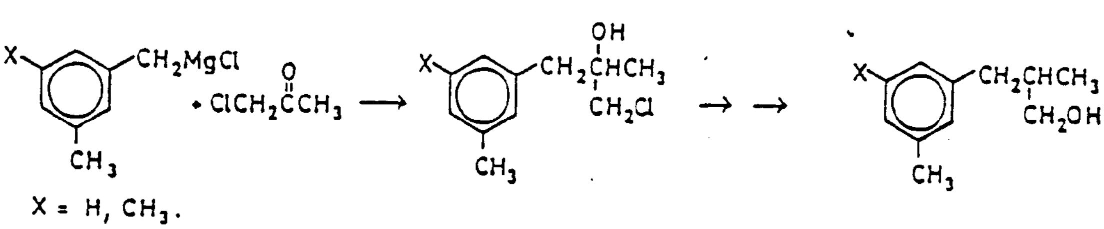

X = H, $CH_3$.

Die als Ausgangsverbindungen verwendeten Benzaldehyde, Propionaldehyd, Benzylchloride und Chloraceton sind bekannte Substanzen. Die Benzylmagnesiumchloride sind in an sich bekannter Weise aus metallischem Magnesium und den Benzylchloriden erhältlich.

Die Umsetzung gemäß a) erfolgt vorzugsweise mit Alkalimetallhydroxiden insbesondere Natriumhydroxid oder Kaliumhydroxid bei Temperaturen von vorzugsweise -5°C bis 80°C in polaren Lösungsmitteln wie Alkoholen beispielsweise Methanol oder Ethanol. Das Reaktionsprodukt gemäß a) wird dann mit Reduktionskatalysatoren wie Palladium auf Aktivkohle oder Raney-Nickel und Wasserstoff hydriert und schließlich durch fraktionierende Destillation der Alkohol separiert.

Die Umsetzung gemäß a1) erfolgt vorzugsweise bei Temperaturen von 0°C bis 60°C in trockenen inerten Lösungsmitteln wie Diethylether und/oder Tetrahydrofuran mit nachfolgender Hydrolyse am besten in Gegenwart von Mineralsäuren wie Salzsäure, Schwefelsäure und dergl. Das Reaktionsprodukt gemäß a1) wird nachfolgend mit Basen vorzugsweise Alkalimetallhydroxyden wie Natriumhydroxyd oder Kaliumhydroxyd bei Temperaturen von vorzugsweise 0°C bis 50°C zum Epoxid cyclisiert, dann katalytisch hydriert vorzugsweise mit Reduktionskatalysatoren wie Palladium auf Aktivkohle und Wasserstoff und schließlich durch fraktionierende Destillation der Alkohol separiert.

Ein weiteres Verfahren zur Herstellung von 2-Methyl-3-(3-methylphenyl)-propan-1-ol und 2-Methyl-3-(3,5-dimethylphenyl)-propan-1-ol ist dadurch gekennzeichnet, daß

a2) 3-Methylbenzylchlorid und 3,5-Dimethylbenzylchlorid mit Methylmalonsäurediethylester umgesetzt wird,

b2) das Reaktionsprodukt gemäß a2) nach erfolgter Verseifung thermisch decarboxyliert wird und

c2) das Reaktionsprodukt gemäß b2) hydriert wird, oder

a3) 3-Methylbenzaldehyd oder 3,5-Dimethylbenzaldehyd mit 2-Brompropionsäuremethylester umgesetzt wird und nach erfolgter Wasserabspaltung

b3) das Reaktionsprodukt gemäß a3) hydriert wird.

Diese Reaktionswege werden an folgenden Schemata verdeutlicht.

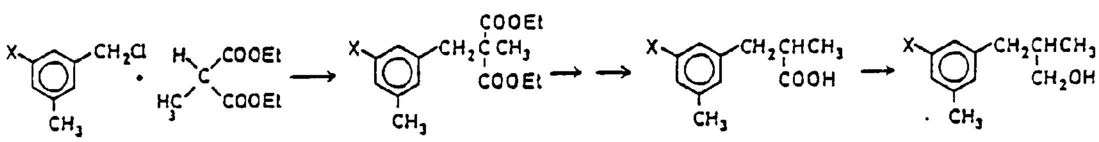

oder

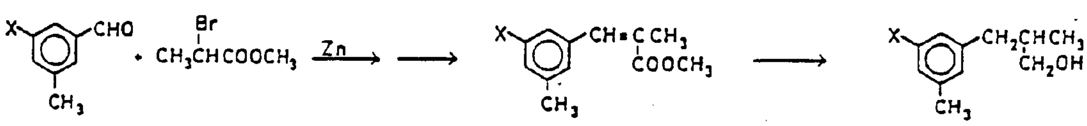


X = H, $CH_3$.

Methylmalonsäurediethylester und 2-Brompropionsäuremethylester sind bekannte Verbindungen. Die Umsetzung gemäß a2) erfolgt vorzugsweise bei Temperaturen von 70 bis 120°C in Gegenwart einer Base wie Natrium- oder Kaliumcarbonat. Nach erfolgter Verseifung beispielsweise mit Basen wie Natriumhydroxyd oder Kaliumhydroxyd wird thermisch decarboxyliert vorzugsweise bei 120 bis 150°C. Die erhaltene Säure oder das Veresterungsprodukt mit Alkoholen wie Methanol oder Ethanol wird mit Hydrierungsmitteln wie Lithiumaluminiumhydrid oder Wasserstoff in Gegenwart eines geeigneten Katalysators umgesetzt.

Die Umsetzung gemäß a3) erfolgt vorzugsweise bei Temperaturen von 80 bis 110°C in Gegenwart von metallischem Zink. Nach erfolgter Wasserabspaltung wird der erhaltene Ester mit Hydriermitteln wie Lithiumaluminiumhydrid oder Wasserstoff in Gegenwart eines geeigneten Katalysators umgesetzt.

Verfahren zur Herstellung von 1-(3-Methylphenyl)-2-methylbutan-3-ol und 1-(3,5-Dimethylphenyl)-2-methylbutan-3-ol sind dadurch gekennzeichnet, daß

2-Methyl-3-(3-methylphenyl)-propanal oder 2-Methyl-3-(3,5-dimethylphenyl)-propanal mit Methylmagnesiumhalogenid (Halogen=Cl,Br,I) umgesetzt wird

oder

3-Methylbenzylchlorid oder 3,5-Dimethylbenzylchlorid mit Methylethylketon in einem organischen/alkalischen 2-Phasensystem in Gegenwart eines Phasentransferkatalysators umgesetzt wird.

Methylhalogenide und Methylethylketon sind bekannt. Die Methylmagnesiumhalogenide sind in an sich bekannter Weise aus metallischem Magnesium und den Methylhalogeniden erhältlich. Die Umsetzung der Propanale mit dem Methylmagnesiumhalogenid erfolgt vorzugsweise bei Temperaturen von 0 bis 50°C in trockenem inertem Lösungsmittel wie Diethylether und/oder Tetrahydrofuran mit nachfolgender Hydrolyse am besten in Gegenwart von Mineralsäuren wie Salzsäure, Schwefelsäure und dgl.

Bei der Umsetzung von Benzylchlorid mit Methylethylketon werden in etwa äquimolare Mengen, oder Methylethylketon im Überschuß eingesetzt. Das organisch/ alkalische 2-Phasensystem wird gebildet aus einem organischen mit Wasser nicht mischbaren inerten Lösungsmittel und einer 5-50 Gew.-%igen wäßrigen Lösung von Alkalimetallhydroxyd oder in fester Form vorliegendem Alkalimetallhydroxyd. Beispiele für Alkalimetallhydroxyde sind Natrium- oder Kaliumhydroxyd. Als Phasentransferkatalysatoren werden beispielsweise Kronenether, quaternäre Ammonium- oder Phosphoniumsalze in Mengen von 0,5-5 Mol-% bezogen auf Benzylchlorid eingesetzt.
Bezüglich weiterer Einzelheiten wird ausdrücklich auf die Beispiele verwiesen.
Die erfindungsgemäßen Verbindungen finden Verwendung als Duftstoffe.
Die erfindungsgemäßen Alkohole haben aufgrund ihrer Beständigkeit gegen Luft und Laugen ein sehr weites Anwendungsgebiet. Neben dem Einsatz in der Parfümerie eignen sie sich auch zur Beduftung von Wasch- und Reinigungsmitteln, Textilien, Kunststofferzeugnissen u.a.

Beispiel 1

2-Methyl-3-(3-methylphenyl)-propan-1-ol

In einem 1l-Vierhalskolben wurden unter Rühren und Argonspülung 7,5g KOH in 240ml Ethanol gelöst. Unter Eiskülung wurden zunächst 123g 3-Methylbenzaldehyd, dann im Verlauf von ca. 4 Stunden 62g Propionaldehyd zugegeben. Die Reaktionstemperatur betrug 8°C. Nachfolgend wurde der Ansatz mit Eisstückchen, enthärtetem Wasser und Ether versetzt. Die untere Schicht wurde verworfen, die obere mit 300-400ml Wasser gewaschen und anschließend über eine 20cm lange Kolonne mit 7mm Glaswendeln destilliert. Es wurden 116g 2-(3- Methylbenzyliden)-propionaldehyd bei 80-90°C und 0,2mbar erhalten. 35g dieser Verbindung, 100ml Cyclohexan und 1g Hydrierkatalysator (5% Pd auf Aktivkohle) wurden in einen 0,5l Schüttelautoklaven gebracht. Bei 25°C wurden 70bar Wasserstoff aufgepreßt und 7 Stunden bei 40°C geschüttelt. Destillation über eine Drehbandkolonne ergab 15g 2-Methyl-3-(3-methylphenyl)-propan-1-ol (Siedepunkt 70°C bei 0,1mbar).

Beispiel 2

2-Methyl-3-(3,5- dimethylphenyl)-propan-1-ol

Es wurden 25g Magnesiumspäne und 155g 3,5-Dimethylbenzylchlorid in 300ml Ether umgesetzt. Nach Verdünnung mit 200ml Tetrahydrofuran und unter Kühlung mit Eis ließ man im Verlauf einer Stunde eine Mischung von 92,5g Chloraceton und 100ml Tetrahydrofuran zutropfen. Nach 2 Stunden Reaktion bei 25°C wurde der Ansatz auf eine Mischung von Eis und 2,5mol Salzsäure gegossen. Die obere Schicht wurde abgetrennt, die wässerige ausgeethert. Die vereinigten organischen Schichten wurden mit Wasser neutral gewaschen, mit $Na_2SO_4$ getrocknet, filtriert und destilliert. Bei 110-112°C/0,2mbar erhielt man 110g 1-Chlor-2-(3,5-dimethylbenzyl)-propan-2-ol.
109g dieser Verbindung wurden mit 200ml Methyl-tert.-butylether und einer Lösung von 31g NaOH in 750ml Wasser 2 Stunden bei 30°C gerührt. Dann wurden die Schichten getrennt, die organische Phase mit Wasser neutral gewaschen, mit $Na_2SO_4$ getrocknet und destilliert. Bei einem Siedepunkt von 68-70°C/0,3mbar wurden 65g 2-(3,5-Dimethylbenzyl)-propylenoxid erhalten.
80g dieser Verbindung wurden in 100ml Cyclohexan gelöst und mit 0,8g 58 Pd auf Aktivkohle in einen 0,5l Schüttelautoklaven gebracht. Nach Spülen mit Inertgas wurde Wasserstoff aufgepreßt (130bar) und 8 Stunden auf 150°C erwärmt. Die erkaltete Mischung wurde entspannt, mit Inertgas gespült, vom Katalysator abfiltriert und über eine 30cm lange Kolonne mit Stahlwendeln destilliert. Bei 80-81°C/0,07mbar gingen 18g 2-Methyl-3-(3,5-dimethylphenyl)-propan-1-ol über.

Beispiel 3

2-Methyl-3-(3-methylphenyl)-propan-1-ol

In einem 2l Vierhalskolben wurden 500ml Toluol, 207g gemahlenes Kaliiumcarbonat, 8g Kaliumiodid, 7g 18-Krone-6 und 209g Methylmalonsäurediethylester vorgelegt. Die Mischung wurde unter Rühren auf 85°C erwärmt. Im Verlauf einer halben Stunde wurden 141g 3-Methylbenzylchlorid zugetropft und der Ansatz dann 8 Stunden bei 90°C gerührt. Nach Abkühlung wurden die Salze durch mehrmaliges Ausschütteln mit Wasser entfernt und die organische Schicht destilliert. Bei einem Siedepunkt von 120°C und einem Druck von etwa 0,02mbar wurden 184g 2-Methyl-2-(3-methylbenzyl)-malonsäurediethylester erhalten. Zur Verseifung wurde der Ester mit 60g NaOH, 170ml Wasser und einer Spatelspitze Tetradecyltrimethylammoniumbromid 16 Stunden unter Rückfluß gekocht und gerührt, wobei zwischenzeitlich entstandenes Ethanol abdestilliert wurde. Durch Ansäuern mit Salzsäure wurde die Dicarbonsäure freigesetzt, abgetrennt und zur Decarboxylierung in Xylol 4 Stunden unter Rückfluß erhitzt. Die Monocarbonsäure wurde anschließend mit Ethanol verestert (p-Toluolsulfonsäure als Katalysator, Cyclohexan als Schleppmittel für Wasser). Es wurden 115g 2-(3-Methylbenzyl)-propionsäureethylester (Siedepunkt 70-73°C bei 0,03mbar) erhalten. Zur Hydrierung wurden in einem 2l Vierhalskolben unter Stickstoff 21g $LiAlH_4$ in 800ml Tetrahydrofuran gelöst bzw. suspendiert. Dann wurde unter Eiskühlung eine Mischung von 110g 2-(3-Methylbenzyl)-propionsäureethylester und 100ml Tetrahydrofuran zugetropft. Anschliessend wurde mit Eis und Salzsäure zersetzt, mit Ether extrahiert über $K_2CO_3$ getrocknet und nach Destillation 75g 2- Methyl-3-(3-methylphenyl)-propan-1-ol (Siedepunkt 68-70°C bei 0,1mbar) erhalten.
Geruchsbild: wäßrig-blumig, grün-fruchtig

Beispiel 4

2-Methyl-3-(3,5-dimethylphenyl)-propan-1-ol

Beispiel 3 wurde mit 3,5-Dimethylbenzylchlorid wiederholt. Es wurden 2-Methyl-3-(3,5-dimethylphenyl)-propan-1-ol (Siedepunkt 83°C bei 0,1mbar) erhalten.
Geruchsnote: rosig, grün

Beispiel 5

1-(3-Methylphenyl)-2-methylbutan-3-ol

256g Methylethylketon, 80g gepulvertes NaOH, 28g Calciumoxid und 10g Tetrabutylammoniumbromid wurden unter Rühren erwärmt. Bei 70°C ließ man im Verlauf von 90 Minuten 141g 3-Methylbenzylchlorid zutropfen. Anschließend wurde 1 Stunde unter Rückfluß gekocht, abgekühlt, filtriert, mit Methylethylketon nachgewaschen, das Filtrat eingeengt und über eine 30cm lange Kolonne mit Stahlwendeln destilliert. Bei einem Siedepunkt von 80°C bei 0,4mbar wurde 86 %-iges 3-(3-Methylbenzyl)-butanon mit 40 % Ausbeute erhalten. Zu 100g mit 300ml Isopropanol zum Sieden erhitztem Aluminiumtriisopropylat wurde binnen 5 Stunden eine Lösung von 1mol 3- (3-Methylbenzyl)-butanon (86 %-ig) in 300ml Isopropanol zugetropft, während über eine 50cm lange Füllkörperkolonne langsam ein Gemisch aus Aceton und Isopropanol abdestillierte. Nach 8 Stunden Reaktion enthielt das Destillat kein Aceton mehr. Nun wurde das restliche Isopropanol abdestilliert und der Rückstand mit 300ml Toluol, 300g Eis und 300g konz. Salzsäure versetzt. Die Schichten wurden getrennt, die wäßrige Phase mit 300ml Toluol extrahiert, die vereinigen organischen Phasen mit wenig Wasser ausgeschüttelt, eingeengt und über eine Vigreux-Kolonne bei 0,2mbar destilliert. Von 78-80°C erhielt man in 85% Ausbeute ein Gemisch von 1-(3- Methylphenyl)-2-methylbutan-3-ol-Isomeren.
Geruchsbild: grün-rosig mit Lindenblüten und Maiglöckchen etwas ambriert.

## Patentansprüche

1. 2-Methyl-3-(3-methylphenyl)-propan-1-ol, 2-Methyl-3-(3,5-dimethylphenyl)-propan-1-ol, 1-(3-Methylphenyl)-2-methylbutan-3-ol und 1-(3,5-Dimethylphenyl)-2-methylbutan-3-ol.

2. Verfahren zur Herstellung von 2-Methyl-3-(3-methylphenyl)-propan-1-ol und 2-Methyl-3-(3,5-dimethylphenyl)-propan-1-ol, dadurch gekennzeichnet, daß

a) 3-Methylbenzaldehyd oder 3,5-Dimethylbenzaldehyd mit Propionaldehyd in Gegenwart von Basen umgesetzt wird und

b) das Reaktionsprodukt gemäß a) hydriert wird.

3. Verfahren zur Herstellung von 2-Methyl-3-(3-methylphenyl)-propan-1-ol und 2-Methyl-3-(3,5- dimethylphenyl)-propan-1-ol, dadurch gekennzeichnet, daß

a1) 3-Methylbenzylmagnesiumchlorid oder 3,5-Dimethylbenzylmagnesiumchlorid mit Chloraceton umgesetzt wird und

b1) das Reaktionsprodukt gemäß a1) nach basenkatalysierter Cyclisierung katalytisch hydriert wird.

4. Verfahren zur Herstellung von 2-Methyl-3-(3-methylphenyl)-propan-1-ol und 2-Methyl-3-(3,5-dimethylphenyl)-propan-1-ol, dadurch gekennzeichnet, daß

a2) 3-Methylbenzylchlorid oder 3,5-Dimethylbenzylchlorid mit Methylmalonsäurediethylester umgesetzt wird,

b2) das Reaktionsprodukt gemäß a2) nach erfolgter Verseifung thermisch decarboxyliert wird und
c2) das Reaktionsprodukt gemäß b2) hydriert wird.
5. Verfahren zur Herstellung von 2-Methyl-3-(3-methylphenyl)-Propan-1-ol und 2-Methyl-3-(3,5-dimethylphenyl)-propan-1-ol, dadurch gekennzeichnet, daß
a3) 3-Methylbenzaldehyd oder 3,5-Dimethylbenzaldehyd mit Zink und 2-Brompropionsäuremethylester umgesetzt wird und nach erfolgter Wasserabspaltung
b3) das Reaktionsprodukt gemäß a3) hydriert wird.
6. Verfahren zur Herstellung von 1-(3-Methylphenyl)-2-methylbutan-3-ol und 1-(3,5-Dimethylphenyl)-1-methylbutan-3-ol, dadurch gekennzeichnet, daß 2-Methyl-3-(3-methylphenyl)-propanal oder 1-Methyl-3-(3,5-dimethylphenyl)-propanal mit Methylmagnesiumhalogenid (Halogen=Cl, Br,I) umgesetzt wird.
7. Verfahren zur Herstellung von 1-(3-Methylphenyl)-2-methylbutan-3-ol und 1-(3,5-Dimethylphenyl)-2-methylbutan-3-ol, dadurch gekennzeichnet, daß 3-Methylbenzylchlorid oder 3,5-Dimethylbenzylchlorid mit Methylethylketon in einem organischen/alkalischen 2-Phasensystem in Gegenwart von Phasentransferkatalysator umgesetzt wird und das jeweilige, erhaltene Butanon-3 mit Aluminiumtriisopropylat reduziert wird.
8. Verwendung der Verbindungen nach Anspruch 1 als Duftstoffe.

## Claims

1. 2-Methyl-3-(3-methylphenyl)-propan-1-ol, 2-methyl-3-(3,5-dimethylphenyl)-propan-1-ol, 1-(3-methylphenyl)-2-methylbutan-3-ol and 1-(3,5-dimethylphenyl)-2-methylbutan-3-ol.
2. Process for the preparation of 2-methyl-3-(3-methylphenyl)-propan-1-ol and 2-methyl-3-(3,5-dimethylphenyl)-propan-1-ol, characterised in that
a) 3-methylbenzaldehyde or 3,5-dimethylbenzaldehyde is reacted with propionaldehyde in the presence of bases and
b) the reaction product from a) is hydrogenated.
3. Process for the preparation of 2-methyl-3-(3-methylphenyl)-propan-1-ol and 2-methyl-3-(3,5-dimethylphenyl)-propan-1-ol, characterised in that
a1) 3-methylbenzylmagnesium chloride or 3,5-dimethylbenzylmagnesium chloride is reacted with chloroacetone and
b1) the reaction product from a1) is catalytically hyrogenated after base-catalysed cyclisation.
4. Process for the preparation of 2-methyl-3-(3-methylphenyl)-propan-1-ol and 2-methyl-3-(3,5-dimethylphenyl)-propan-1-ol, characterised in that
a2) 3-methylbenzyl chloride or 3,5-dimethylbenzyl chloride is reacted with diethyl methylmalonate,
b2) the reaction product from a2) is thermally decarboxylated after hydrolysis and
c2) the reaction product from b2) is hydrogenated.
5. Process for the preparation of 2-methyl-3-(3-methylphenyl)-propan-1-ol and 2-methyl-3-(3,5-dimethylphenyl)-propan-1-ol, characterised in that
a3) 3-methylbenzaldehyde or 3,5-dimethylbenzaldehyde is reacted with zinc and methyl 2-bromopropionate and, after elimination of water,
b3) the reaction product from a3) is hydrogenated.
6. Process for the preparation of 1-(3-methylphenyl)-2-methylbutan-3-ol and 1-(3,5-dimethylphenyl)-2-methylbutan-3-ol, characterised in that 2-methyl-3-(3-methylphenyl)-propanal or 2-methyl-3-(3,5-dimethylphenyl)-propanal is reacted with methylmagnesium halide (halogen = Cl, Br, I).
7. Process for the preparation of 1-(3-methylphenyl)-2-methylbutan-3-ol and 1-(3,5-dimethylphenyl)-2-methylbutan-3-ol, characterised in that 3-methylbenzyl chloride or 3,5-dimethylbenzyl chloride is reacted with methyl ethyl ketone in an organic/alkaline two-phase system in the presence of phase transfer catalyst and the respective butan-3-one obtained is reduced with aluminum triisopropylate.
8. Use of the compounds according to Claim 1 as fragrances.

## Revendications

1. 2-Méthyl-3-(3-méthylphényl)-propane-1-ol, 2-méthyl-3-(3,5-diméthylphényl)-propane-1-ol, 1-(3-méthylphényl)-2-méthylbutane-3-ol et 1-(3,5-diméthylphényl)-2-méthylbutane-3-ol.
2. Procédé de préparation du 2-méthyl-3-(3-méthylphényl)-propane-1-ol et du 2-méthyl-3-(3,5-diméthylphényl)-propane-1-ol, procédé caractérisé en ce que :
a) on fait réagir le 3-méthylbenzaldéhyde ou le 3,5-diméthylbenzaldéhyde avec le propionaldéhyde en présence d'une base et

b) on hydrogène le produit ainsi formé.

3. Procédé de préparation du 2-méthyl-3-(3-méthylphényl)-propane-1-ol et du 2-méthyl-3-(3,5-diméthylphényl)-propane-1-ol, procédé caractérisé en ce que :

a1) on fait réagir le chlorure de 3-méthylbenzyl-magnésium ou de 3,5-diméthylbenzyl-magnésium avec la chloracétone et

b1) après cyclisation catalysée avec une base on hydrogène catalytiquement le produit ainsi formé.

4. Procédé de préparation du 2-méthyl-3-(3-méthylphényl)-propane-1-ol et du 2-méthyl-3-(3,5-diméthylphényl)-propane-1-ol, procédé caractérisé en ce que :

a2) on fait réagir le chlorure de 3-méthylbenzyle ou de 3,5-diméthylbenzyle avec le méthylmalonate de diéthyle,

b2) après l'avoir saponifié on décarboxyle sous l'action de la chaleur le produit ainsi obtenu, puis

c2) on hydrogène le produit formé en b2).

5. Procédé de préparation du 2-méthyl-3-(3-méthylphényl)-propane-1-ol et du 2-méthyl-3-(3,5-diméthylphényl)-propane-1-ol, procédé caractérisé en ce que :

a3) on fait réagir le 3-méthylbenzaldéhyde ou le 3,5-diméthylbenzaldéhyde avec du zinc et le 2-bromopropionate de méthyle, et après avoir séparé l'eau formée

b3) on hydrogène le produit obtenu en a3.

6. Procédé de préparation du 1-(3-méthylphényl)-2-méthylbutane-3-ol et du 1-(3,5-diméthylphényl)-2-méthylbutane-3-ol, procédé caractérisé en ce que l'on fait réagir le 2-méthyl-3-(3-méthylphényl)-propanal ou le 2-méthyl-3-(3,5-diméthylphényl)-propanal avec un halogénure de méthylmagnésium dont l'halogène peut être le chlore, le brome ou l'iode.

7. Procédé de préparation du 1-(3-méthylphényl)-2-méthylbutane-3-ol et du 1-(3,5-diméthylphényl)-2-méthylbutane-3-ol, procédé caractérisé en ce que l'on fait réagir le chlorure 3-méthylbenzyle ou de 3,5-diméthylbenzyle avec la méthyléthylcétone dans un milieu à deux phases hydroalcalin, en présence d'un catalyseur de transfert de phases, puis dans les deux cas on réduit avec du tri-isopropylate d'aluminium la butanone-3 obtenue.

8. L'emploi comme parfums des composés de la revendication 1.